# EUROPEAN PATENT SPECIFICATION

(11) **EP 2 234 984 B1**
(45) Date of publication and mention of the grant of the patent: **16.03.2016**
(21) Application number: 08862464.8
(22) Date of filing: 16.12.2008
(51) Int. Cl.: C07D 235/08, C07D 401/12, C07D 403/12, A61K 31/4184

(54) **FLUOROALKYL SUBSTITUTED BENZIMIDAZOLE CANNABINOID AGONISTS**
FLUORALKYLSUBSTITUIERTE BENZIMIDAZOLE ALS CANNABINOID-AGONISTEN
BENZIMIDAZOLE SUBSTITUÉES PAR FLUOROALKYLE COMME AGONISTES CANNABINOÏDES

(30) Priority: 17.12.2007 EP 07123371
(43) Date of publication of application: 06.10.2010
(73) Proprietor: Janssen Pharmaceutica, N.V., 2340 Beerse (BE)
(72) Inventor: GIJSEN, Henricus, Jacobus, Maria, B-2340 Beerse (BE); VERBIST, Bie, Maria, Pieter, B-2340 Beerse (BE); SURKYN, Michel, B-2340 Beerse (BE)
(74) Representative: Verberckmoes, Filip Gerard
(86) International application number: PCT/EP2008/067645
(87) International publication number: WO 2009/077533

(56) References cited:
- WO-A-2006/048754
- WO-A-2007/102059
- WO-A-2008/003665

## Description

The present invention is related to novel benzimidazole compounds of formula (I) having selective cannabinoid receptor 2 agonistic properties, pharmaceutical compositions comprising these compounds, chemical processes for preparing these compounds and these compounds for use in the treatment of diseases linked to the mediation of the cannabinoid receptors in animals, in particular humans.

Classical cannabinoids such as the marijuana derived cannabinoid Δ⁹-tetrahydro-cannabinol, (Δ⁹-THC) produce their pharmacological effects via interaction with specific cannabinoid receptors in the body. So far, two cannabinoid receptors have been characterized : CB1, a receptor found in the mammalian brain and peripheral tissues and CB2, a receptor found predominantly in the peripheral tissues. Compounds that are agonists or antagonists for one or both of these receptors have been shown to provide a variety of pharmacological effects. There is considerable interest in developing cannabinoid analogs that have selective CB2 agonistic activity since it is believed selectivity for CB2 receptor may offer avenues for harnessing the beneficial effect of CB receptor agonists while avoiding the central adverse events seen with cannabinoid structures (see e.g. Expert Opinion on Investigational Drugs (2005), 14(6), 695-703). On the other hand, a certain level of CB1 agonistic acivity in the compounds can offer an additional increase in beneficial effects of the agonists. A good balance between CB1 and CB2 agonistic properties, preferably combined with a low central (brain) penetration of the compounds may therefore offer a clinical advantage over the use of completely selective CB2 agonists.

WO-2002/46168 discloses benzimidazole compounds as estrogen receptor-β ligands for use in the treatment of diseases related to the estrogen receptor-β such as Alzheimer's disease, anxiety disorders, depressive disorders, osteoporosis, cardiovascular disease, rheumatoid arthritis or prostate cancer. WO-2006/048754 and WO-2007/102059 disclose sulfonyl benzimidazole derivatives having CB2 agonistic activity useful in the treatment of conditions mediated by CB2 receptor activity.

The compounds of the present invention differ structurally from the cited art known compounds by the presence of a trifluoro substituted alkyl group on the benzimidazole moiety (especially WO-2006/048754) or a different substitution pattern of the sulfonyl substituent (compared to WO-2007/102059).

The present invention relates to a compound of formula (I) 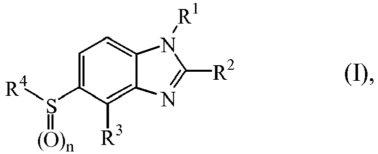 including any stereochemically isomeric form thereof, wherein
n is an integer 0, 1 or 2;
R¹ is n-trifluorobutyl or n-trifluoropentyl;
R² is C₁₋₆alkyl;
R³ is hydrogen;
R⁴ is C₁₋₈alkyl substituted with C₃₋₈cycloalkyl;
   C₁₋₈alkyl substituted with 1, 2 or 3 substituents each independently selected from halo, C₁₋₈alkyl, or aryl;
   heterocyclyl;
   aryl; or
   heteroaryl;
heterocyclyl is selected from pyrrolidinonyl;
aryl is phenyl substituted with 1 substituent selected from C₁₋₄alkyloxy, cyano, or R⁷-carbonyl; wherein R⁷ is amino;
heteroaryl is selected from unsubstituted pyrazolyl or unsubstituted pyridinyl or a pharmaceutically acceptable acid addition salt thereof;
provided that 2-tert-butyl-5-[(4-methoxybenzyl)sulfanyl]-1-(4,4,4-trifluorobutyl)-1H-benzimidazole is not included.

As used in the foregoing definitions :
- halo is generic to fluoro, chloro, bromo and iodo;
- C₁₋₄alkyl defines straight and branched chain saturated hydrocarbon radicals having from 1 to 4 carbon atoms such as, for example, methyl, ethyl, propyl, butyl, 1-methylethyl, 2-methylpropyl and the like;
- C₁₋₆alkyl is meant to include C₁₋₄alkyl and the higher homologues thereof having 5 or 6 carbon atoms, such as, for example, 2-methylbutyl, pentyl, hexyl and the like;
- C₃₋₆alkyl defines straight and branched chain saturated hydrocarbon radicals having from 3 to 6 carbon atoms such as, for example, propyl, butyl, pentyl, hexyl, 1-methylethyl, 2-methylpropyl, 2-methylbutyl and the like;
- polyhaloC₃₋₆alkyl is defined as polyhalosubstituted C₃₋₆alkyl (as hereinabove defined) substituted with 2 to 6 halogen atoms such as difluoromethyl, trifluoromethyl, trifluoroethyl, n-trifluorobutyl or n-trifluoropentyl and the like;
- C₃₋₆cycloalkyl is generic to cyclopropyl, cyclobutyl, cyclopentyl, and cyclohexyl;
- C₃₋₈cycloalkyl is generic to cyclopropyl, cyclobutyl, cyclopentyl, cyclohexyl, cycloheptyl and cyclooctyl;
- C₆₋₈cycloalkyl is generic to cyclohexyl, cycloheptyl and cyclooctyl.

The term "stereochemically isomeric forms" as used hereinbefore defines all the possible isomeric forms which the compounds of formula (I) may possess. Unless otherwise mentioned or indicated, the chemical designation of compounds denotes the mixture of all possible stereochemically isomeric forms, said mixtures containing all diastereomers and enantiomers of the basic molecular structure. More in particular, stereogenic centers may have the R- or S-configuration; substituents on bivalent cyclic (partially) saturated radicals may have either the cis- or trans-configuration. Stereochemically isomeric forms of the compounds of formula (I) are obviously intended to be embraced within the scope of this invention.

The absolute stereochemical configuration of the compounds of formula (I) and of the intermediates used in their preparation may easily be determined by those skilled in the art while using well-known methods such as, for example, X-ray diffraction.

Furthermore, some compounds of formula (I) and some of the intermediates used in their preparation may exhibit polymorphism. It is to be understood that the present invention encompasses any polymorphic forms possessing properties useful in the treatment of the conditions noted hereinabove.

The pharmaceutically acceptable acid addition salts as mentioned hereinabove are meant to comprise the therapeutically active non-toxic acid addition salt forms that the compounds of formula (I) are able to form. These pharmaceutically acceptable acid addition salts can conveniently be obtained by treating the base form with such appropriate acid. Appropriate acids comprise, for example, inorganic acids such as hydrohalic acids, e.g. hydrochloric or hydrobromic acid, sulfuric, nitric, phosphoric and the like acids; or organic acids such as, for example, acetic, propanoic, hydroxyacetic, lactic, pyruvic, oxalic (*i.e.* ethanedioic), malonic, succinic (*i.e.* butanedioic acid), maleic, fumaric, malic, tartaric, citric, methanesulfonic, ethanesulfonic, benzenesulfonic, *p*-toluenesulfonic, cyclamic, salicylic, *p*-aminosalicylic, pamoic and the like acids.

Conversely said salt forms can be converted by treatment with an appropriate base into the free base form.

The compounds of formula (I) may exist in both unsolvated and solvated forms. The term 'solvate' is used herein to describe a molecular association comprising a compound of the invention and one or more pharmaceutically acceptable solvent molecules, e.g. water or ethanol. The term 'hydrate' is used when said solvent is water.

Interesting compounds of formula (I) are those compounds of formula (I) wherein one or more of the following restrictions apply :
a) n is an integer 0, or n is an integer 2; or
c) R¹ is n-trifluorobutyl or n-trifluoropentyl; or
d) R² is C₁₋₆alkyl, in particular R² is *tert*-butyl; or
e) R³ is hydrogen; or
f) R⁴ is C₁₋₈alkyl substituted with C₃₋₈cycloalkyl; or
f) R⁴ is C₁₋₈alkyl substituted with heteroaryl wherein heteroaryl is unsubstituted pyridinyl or unsubstituted pyrazolyl.

Compounds of formula (I-a), defined as compounds of formula (I) wherein n is 0, can be prepared by reacting intermediate (II) with an intermediate (III), wherein L is a leaving group such as halo, methanesulfonyloxy, benzenesulfonyloxy, trifluoromethanesulfonyloxy and the like reactive leaving groups, in the presence of a suitable base such as Cs₂CO₃ in a reaction-inert solvent such as e.g. 2-propanone, 1,4-dioxane or THF, and optionally in the presence of NaI or KI. Depending upon the type of substituents present in intermediate (III) it may be necessary to introduce protecting groups in intermediate (III) which can be removed after the coupling reaction.

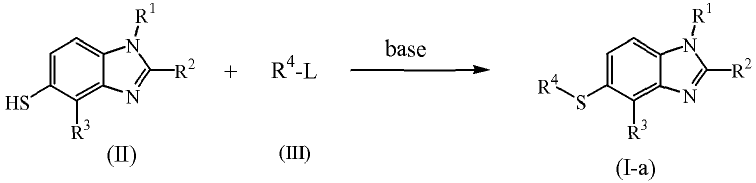

Compounds of formula (I-a), defined as compounds of formula (I) wherein n is 0, can also be prepared by reacting intermediate (II) with an intermediate (IV) in the presence of a suitable base such as Cs₂CO₃, a catalyst such as Pd₂(dba)₃ and a suitable ligand such as Xantphos, in a reaction-inert solvent such as e.g. 2-propanone, 1,4-dioxane or THF.

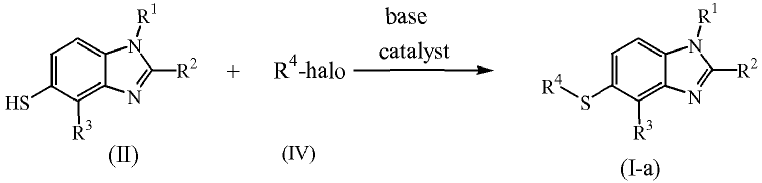

Compounds of formula (I-a) can be converted into compounds of formula (I-b), defined as compounds of formula (I) wherein n represents 1, or into compounds of formula (I-c), defined as compounds of formula (I) wherein n represents 2, by art known S-oxidation reactions.

S-oxidation reactions can be performed using a 30% aqueous solution of hydrogen peroxide, or by other oxidizing agents such as, NaIO₄, *tert*-butyloxychloride, acyl nitrites, sodium perborate and peracids such as mCPBA (meta-chloroperbenzoic acid). Sulfides can be oxidized to sulfoxides which can be further oxidized to sulfones by addition of another equivalent of hydrogen peroxide, KMnO₄, sodium perborate, potassium hydrogen persulfate, mCPBA or the like reagents. If enough oxidizing agent is present, sulfides can be converted directly to sulfones without isolation of the sulfoxides.

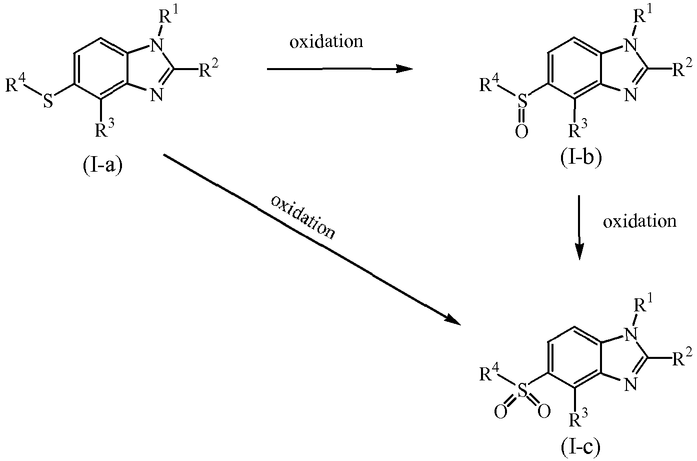

The compounds of formula (I) as prepared in the hereinabove described processes may be synthesized in the form of racemic mixtures of enantiomers which can be separated from one another following art-known resolution procedures. Those compounds of formula (I) that are obtained in racemic form may be converted into the corresponding diastereomeric salt forms by reaction with a suitable chiral acid. Said diastereomeric salt forms are subsequently separated, for example, by selective or fractional crystallization and the enantiomers are liberated therefrom by alkali. An alternative manner of separating the enantiomeric forms of the compounds of formula (I) involves liquid chromatography using a chiral stationary phase. Said pure stereochemically isomeric forms may also be derived from the corresponding pure stereochemically isomeric forms of the appropriate starting materials, provided that the reaction occurs stereospecifically. Preferably if a specific stereoisomer is desired, said compound will be synthesized by stereospecific methods of preparation. These methods will advantageously employ enantiomerically pure starting materials.

The compounds of formula (I), the pharmaceutically acceptable salts and stereoisomeric forms thereof possess selective cannabinoid recepter 2 (CB2) agonistic properties as demonstrated in the Pharmacological Examples. Pharmacological example D.1 describes the methodology to measure CB2 agonism and results are listed in Table D.1.

Therefore the present compounds of formula (I) are useful as a medicine especially in the treatment of a condition or disease mediated by the cannabinoid 2 receptor, in particular CB2 agonistic activity. Subsequently the present compounds may be used for the manufacture of a medicine for treatment of a condition or a disease mediated by CB2 receptor activity, in particular CB2 agonistic activity.

Preferably, the present invention also provides the use of a compound of formula (I) or a pharmaceutically acceptable salt thereof for the manufacture of a medicament for the treatment of conditions or diseases selected from CB2 conditions or diseases.

Further, the present invention provides compounds for use in a method of treatment of a condition mediated by CB2 receptor activity, in a mammalian subject, which comprises administering to a mammal in need of such treatment a therapeutically effective amount of a compound of formula (I) or a pharmaceutically acceptable salt thereof.

Cannabinoid receptor 2 mediated conditions or disorders are e.g. cardiovascular diseases, such as e.g. atherosclerosis, hypertension, myocardial ischemia; chronic pain disorders, such as e.g. hyperalgesia, neuropathic pain, peripheral pain, visceral pain, inflammatory pain, thermal hyperalgesia, nociceptive pain, fibromyalgia, chronic low back pain, and dental pain; inflammation, oedema, bladder inflammation, neuroinflammatory diseases, immune system disorders, autoimmune diseases, multiple sclerosis, rheumatoid arthritis, gastrointestinal disorders, intestinal motility disorders, irritable bowel syndrome (IBS), inflammatory bowel disease (IBD), Crohn's disease, chronic liver injury (cirrhosis), cancer, prostate cancer, cancer pain, glioma, allergy, nausea and vomiting, asthma, chronic obstructive pulmonary diseases, psoriasis, epilepsy, and bone loss disorders, such as e.g., osteoporosis (hereinafter, refereed as 'CB2 disorders or diseases').

The term "treating" and "treatment', as used herein, refers to curative, palliative and prophylactic treatment, including reversing, alleviating, inhibiting the progress of, or preventing the disease, disorder or condition to which such term applies, or one or more symptoms of such disease, disorder or condition.

Additionally the present invention provides pharmaceutical compositions comprising at least one pharmaceutically acceptable carrier and a therapeutically effective amount of a compound of formula (I).

In order to prepare the pharmaceutical compositions of this invention, an effective amount of the particular compound, in base or acid addition salt form, as the active ingredient is combined in intimate admixture with at least one pharmaceutically acceptable carrier, which carrier may take a wide variety of forms depending on the form of preparation desired for administration. These pharmaceutical compositions are desirably in unitary dosage form suitable, preferably, for oral administration, rectal administration, percutaneous administration or parenteral injection.

For example in preparing the compositions in oral dosage form, any of the usual liquid pharmaceutical carriers may be employed, such as for instance water, glycols, oils, alcohols and the like in the case of oral liquid preparations such as suspensions, syrups, elixirs and solutions; or solid pharmaceutical carriers such as starches, sugars, kaolin, lubricants, binders, disintegrating agents and the like in the case of powders, pills, capsules and tablets. Because of their easy administration, tablets and capsules represent the most advantageous oral dosage unit form, in which case solid pharmaceutical carriers are obviously employed. For parenteral injection compositions, the pharmaceutical carrier will mainly comprise sterile water, although other ingredients may be included in order to improve solubility of the active ingredient. Injectable solutions may be prepared for instance by using a pharmaceutical carrier comprising a saline solution, a glucose solution or a mixture of both. Injectable suspensions may also be prepared by using appropriate liquid carriers, suspending agents and the like. In compositions suitable for percutaneous administration, the pharmaceutical carrier may optionally comprise a penetration enhancing agent and/or a suitable wetting agent, optionally combined with minor proportions of suitable additives which do not cause a significant deleterious effect to the skin. Said additives may be selected in order to facilitate administration of the active ingredient to the skin and/or be helpful for preparing the desired compositions. These topical compositions may be administered in various ways, e.g., as a transdermal patch, a spot-on or an ointment. Addition salts of the compounds of formula (I), due to their increased water solubility over the corresponding base form, are obviously more suitable in the preparation of aqueous compositions.

It is especially advantageous to formulate the pharmaceutical compositions of the invention in dosage unit form for ease of administration and uniformity of dosage. "Dosage unit form" as used herein refers to physically discrete units suitable as unitary dosages, each unit containing a predetermined amount of active ingredient calculated to produce the desired therapeutic effect in association with the required pharmaceutical carrier. Examples of such dosage unit forms are tablets (including scored or coated tablets), capsules, pills, powder packets, wafers, injectable solutions or suspensions, teaspoonfuls, tablespoonfuls and the like, and segregated multiples thereof.

For oral administration, the pharmaceutical compositions of the present invention may take the form of solid dose forms, for example, tablets (both swallowable and chewable forms), capsules or gelcaps, prepared by conventional means with pharmaceutically acceptable excipients and carriers such as binding agents (e.g. pregelatinised maize starch, polyvinylpyrrolidone, hydroxypropylmethylcellulose and the like), fillers (e.g. lactose, microcrystalline cellulose, calcium phosphate and the like), lubricants (e.g. magnesium stearate, talc, silica and the like), disintegrating agents (e.g. potato starch, sodium starch glycollate and the like), wetting agents (e.g. sodium laurylsulphate) and the like. Such tablets may also be coated by methods well known in the art.

Liquid preparations for oral administration may take the form of e.g. solutions, syrups or suspensions, or they may be formulated as a dry product for admixture with water and/or another suitable liquid carrier before use. Such liquid preparations may be prepared by conventional means, optionally with other pharmaceutically acceptable additives such as suspending agents (e.g. sorbitol syrup, methylcellulose, hydroxypropylmethylcellulose or hydrogenated edible fats), emulsifying agents (e.g. lecithin or acacia), non-aqueous carriers (e.g. almond oil, oily esters or ethyl alcohol), sweeteners, flavours, masking agents and preservatives (e.g. methyl or propyl p-hydroxybenzoates or sorbic acid).

Pharmaceutically acceptable sweeteners useful in the pharmaceutical compositions of the invention comprise preferably at least one intense sweetener such as aspartame, acesulfame potassium, sodium cyclamate, alitame, a dihydrochalcone sweetener, monellin, stevioside sucralose (4,1',6'-trichloro-4,1',6'-trideoxygalactosucrose) or, preferably, saccharin, sodium or calcium saccharin, and optionally at least one bulk sweetener such as sorbitol, mannitol, fructose, sucrose, maltose, isomalt, glucose, hydrogenated glucose syrup, xylitol, caramel or honey. Intense sweeteners are conveniently used in low concentrations. For example, in the case of sodium saccharin, the said concentration may range from about 0.04% to 0.1% (weight/volume) of the final formulation. The bulk sweetener can effectively be used in larger concentrations ranging from about 10% to about 35%, preferably from about 10% to 15% (weight/volume).

The pharmaceutically acceptable flavours which can mask the bitter tasting ingredients in the low-dosage formulations are preferably fruit flavours such as cherry, raspberry, black currant or strawberry flavour. A combination of two flavours may yield very good results. In the high-dosage formulations, stronger pharmaceutically acceptable flavours may be required such as Caramel Chocolate, Mint Cool, Fantasy and the like.

Each flavour may be present in the final composition in a concentration ranging from about 0.05% to 1% (weight/volume). Combinations of said strong flavours are advantageously used. Preferably a flavour is used that does not undergo any change or loss of taste and/or color under the circumstances of the formulation.

The compounds of formula (I) may be formulated for parenteral administration by injection, conveniently intravenous, intra-muscular or subcutaneous injection, for example by bolus injection or continuous intravenous infusion. Formulations for injection may be presented in unit dosage form, e.g. in ampoules or multi-dose containers, including an added preservative. They may take such forms as suspensions, solutions or emulsions in oily or aqueous vehicles, and may contain formulating agents such as isotonizing, suspending, stabilizing and/or dispersing agents. Alternatively, the active ingredient may be present in powder form for mixing with a suitable vehicle, e.g. sterile pyrogen-free water, before use.

The compounds of formula (I) may also be formulated in rectal compositions such as suppositories or retention enemas, e.g. containing conventional suppository bases such as cocoa butter and/or other glycerides.

Those of skill in the treatment of diseases linked to the mediation of the cannabinoid receptors will easily determine the therapeutically effective amount of a compound of formula (I) from the test results presented hereinafter. In general it is contemplated that a therapeutically effective dose will be from about 0.001 mg/kg to about 50 mg/kg of body weight, more preferably from about 0.01 mg/kg to about 10 mg/kg of body weight of the patient to be treated. It may be appropriate to administer the therapeutically effective dose in the form of two or more sub-doses at appropriate intervals throughout the day. Said sub-doses may be formulated as unit dosage forms, for example each containing from about 0.1 mg to about 1000 mg, more particularly from about 1 to about 500 mg, of the active ingredient per unit dosage form.

As used herein, a "therapeutically effective amount" of a compound, is the quantity of a compound which, when administered to an individual or animal, results in a sufficiently high level of that compound in the individual or animal to cause a discernible increase or decrease in stimulation of cannabinoid receptors.

The exact dosage and frequency of administration depends on the particular compound of formula (I) used, the particular condition being treated, the severity of the condition being treated, the age, weight and general physical condition of the particular patient as well as the other medication, the patient may be taking, as is well known to those skilled in the art. Furthermore, said "therapeutically effective amount" may be lowered or increased depending on the response of the treated patient and/or depending on the evaluation of the physician prescribing the compounds of the instant invention. The effective daily amount ranges mentioned hereinabove are therefore only guidelines.

### Experimental part

In the procedures described hereinafter the following abbreviations were used: 'CH₂Cl₂' stands for dichloromethane, 'THF' stands for tetrahydrofuran, 'DIPE' stands for diisopropylether, 'NaBH₃(CN)' stands for sodium cyanotrihydroborate, 'Cs₂CO₃' means cesium carbonate, 'MgSO₄' means magnesium sulphate, 'NaHCO₃' means carbonic acid monosodium salt, 'NaOH' means sodium hydroxide, 'Pd₂(dba)₃' means tris[*µ*-[(1,2-*η*:4,5-*η*)-(1E,4E)-1,5-diphenyl-1,4-pentadien-3-one]]dipalladium and 'Xantphos' means (9,9-dimethyl-9H-xanthene-4,5-diyl)bis[diphenylphosphine], 'DMAP' means 4-(dimethylamino)pyridine, 'CF₃COOH' means 2,2,2-trifluoroacetic acid, 'EtOAc' means ethyl acetate, and 'CHCl₃' means trichloromethane. Isolute™ filters are short columns comprising a modified form of diatomaceous earth that can remove water and/or solid impurities from a sample.

High-Performance Liquid Chromatography purification methods :
- Purification method A
   The product was purified by reversed-phase high-performance liquid chromatography (Shandon Hyperprep^{®} C18 BDS (Base Deactivated Silica) 8 µm, 250 g, I.D. 5 cm). A gradient with 2 mobile phases was applied. Phase A: a 0.25 % NH₄HCO₃ solution in water; phase B: CH₃CN).
- Purification method B
   The product was purified by reversed-phase high-performance liquid chromatography (Shandon Hyperprep^{®} C18 BDS (Base Deactivated Silica) 8 µm, 250 g, I.D. 5 cm). A gradient with 3 mobile phases was applied. Phase A: a 0.25 % NH₄HCO₃ solution in water; phase B (optional): CH₃OH; phase C: CH₃CN).

### A. Synthesis of the intermediates

### Example A.1

a) Preparation of 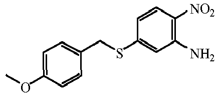 intermediate (1)
   A mixture of 5-chloro-2-nitrobenzenamine (0.16 mol), 4-methoxybenzenemethanethiol (0.16 mol) and potassium hydroxide (0.30 mol) in ethanol (500 ml) was stirred and refluxed for 2 hours. The reaction mixture was cooled. The precipitate was filtered off, washed with ethanol and dried, yielding 48.5 g of intermediate (1).
b) Preparation of 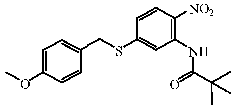 intermediate (2)
   A solution of 2,2-dimethylpropanoyl chloride (0.032 mol) in CH₂Cl₂ (20 ml) was added dropwise to a mixture of intermediate (1) (0.03 mol) and pyridine (0.06 mol) in CH₂Cl₂ (180 ml), cooled on an ice bath. The reaction mixture was allowed to reach room temperature. DMAP was added and the mixture was stirred and refluxed for 20 hours. Extra intermediate (1) (0.01 mol), 2,2-dimethylpropanoyl chloride (0.048 mol) and pyridine (1.2 mol) were added. The mixture was refluxed for 2 hours. The solvent was evaporated. The residue was taken up into CH₂Cl₂ and washed with water. The organic layer was separated, dried (MgSO₄), filtered and the solvent was evaporated. The residue was crystallized from DIPE. The precipitate was filtered off, washed and dried, yielding 9.1 g of intermediate (2).
c) Preparation of 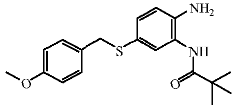 intermediate (3)
   A mixture of intermediate (2) (0.0748 mol), iron (56 g) and acetic acid (10 ml) in water (500 ml) was stirred and refluxed for 4 hours. The mixture was cooled. The solvent was decanted. The residue was taken up into methanol and THF. The mixture was filtered over Dicalite. The solvent was evaporated. The residue was taken up into CH₂Cl₂. The organic layer was separated and filtered over MgSO₄ and Dicalite. The solvent was evaporated. The residue was crystallized from DIPE. The precipitate was filtered off and dried, yielding 21 g of intermediate (3).
d) Preparation of 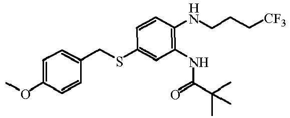 intermediate (4)
   A mixture of intermediate (3) (0.030 mol) and acetic acid (0.0910 mol) in CH₂Cl₂ (500 ml) was stirred at room temperature under nitrogen-bubbling. 4,4,4-Trifluoro-butanal (0.0400 mol) was added. After 15 minutes NaBH₃(CN) was added and the reaction mixture was stirred for another hour. Water was added and the reaction mixture was extracted. The separated organic layers were collected, dried (MgSO₄) and the filtrate was evaporated. The residue was suspended in DIPE, yielding intermediate (4).

### Example A.2

Preparation of 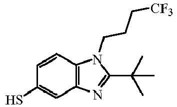 intermediate (5)
Reaction was performed in a microwave. Compound (1) (0.0027 mol) was dissolved in CF₃COOH (15 ml) and the reaction mixture was stirred for 30 minutes at 100°C. Since 10% of the starting material was still present, the mixture was stirred again for 30 minutes at 100°C (microwave). The solvent was evaporated the residue was extracted (EtOAc/NaHCO₃), dried (MgSO₄), filtered and evaporated The concentrate was used as intermediate (5).

Using an analogous procedure, compound (10) was converted into intermediate (9).

Preparation of 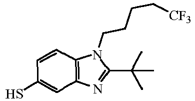 intermediate (9)

### Example A.3

Preparation of 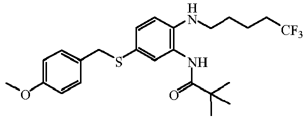 intermediate (8)
5,5,5-Trifluoropentanal (0.150 mol) in CH₂Cl₂ (1000 ml), intermediate (3) (0.050 mol) was added, then acetic acid (4 ml) and Ti(IV)(OiPr)₄ (0.018 mol) were added. The reaction mixture was stirred for 20 minutes. Then NaBH₃(CN) (0.080 mol) was added, the reaction mixture was stirred for 2 hours. The reaction mixture was washed with water. The organic layer was dried (MgSO₄), filtered and evaporated. The residue was purified by column chromatography (400 gram silica column, eluent: CH₂Cl₂). The product fractions were collected and evaporated. The residue solidified upon evaporation. The solid was dried in the oven under vacuum, yielding 12.5 g of intermediate (8).

### Example A.4

Preparation of 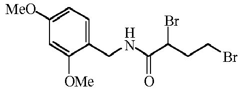 intermediate (10)
To a mixture of 2,4-dimethoxybenzylamine (0.0295 mol) in CH₂Cl₂ (250 mL), cooled at 0°C, was added 2,4-dibromobutyryl bromide. The reaction mixture was allowed to come to room temperature and subsequently stirred at room temperature for 1 hour. The reaction mixture was washed with water and brine. The organic layer was dried (MgSO₄), filtered and evaporated, yielding intermediate (10).

Preparation of 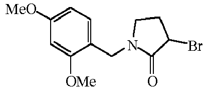 intermediate (11)
To a mixture of intermediate (10) (0.0091 mol) in THF (125 mL), cooled at 0°C, was added NaH (60% in mineral oil, 0.0182 mol). The reaction mixture was stirred at room temperature for 1 hour. The solvent was removed under reduced pressure, and the residue was taken up in CH₂Cl₂ and washed with water. The organic layer was dried (MgSO₄), filtered and evaporated, yielding intermediate (11).

### B. Synthesis of the final compounds

### Example B.1 (Compound (1) is provided only for the purpose of illustration)

Preparation of 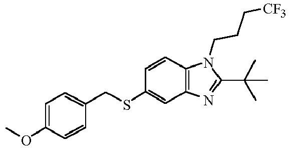 compound (1)
Intermediate (4) (0.0255 mol) was dissolved in acetic acid (100 ml). The reaction mixture was refluxed overnight and the solvent was evaporated. The residue was extracted (CH₂Cl₂/NaHCO₃), dried, filtered and evaporated The concentrate was suspended in DIPE and the precipitate was filtered, yielding 7.4 g of compound (1).

### Example B.2

Preparation of 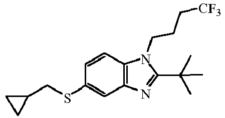 compound (2)
A mixture of (bromomethyl)cyclopropane (0.0057 mol), potassium iodide (catalytic quantity) in THF was stirred for 15 minutes. Intermediate (5) (0.0023 mol) dissolved in THF was added immediately after Cs₂CO₃ (0.0035 mol). The reaction mixture was stirred at 60°C for 1 hour. The mixture was extracted (CH₂Cl₂/H₂O), dried, filtered and evaporated, yielding compound (2).

### Example B.3

Preparation of 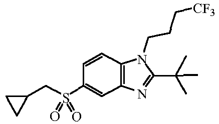 compound (3)
Compound (2) (0.0023 mol) was dissolved in CHCl₃ (40 ml) at room temperature. 3-Chlorobenzenecarboperoxoic acid (0.0054 mol) was added slowly (exothermic reaction) and the mixture was stirred for 1/2 hour. The reaction mixture was extracted twice with saturated NaHCO₃ and once with 1N NaOH. The organic layers were washed with H₂O and dried (MgSO₄). The residue was purified by high-performance liquid chromatography using purification method A. The desired fractions were collected and the solvent was evaporated, yielding compound (3).

### Example B.4

a) Preparation of 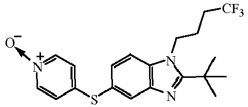 intermediate (6)
   A mixture of 4-chloropyridine 1-oxide (0.0025 mol), Pd₂(dba)₃ (catalytic quantity), Xantphos (catalytic quantity) and Cs₂CO₃ (0.973 g) in dioxane (5 ml) was degassed by applying alternating a nitrogen atmosphere and vacuum. Intermediate (5) (0.0023 mol) in dioxane (15 ml) was added under nitrogen-atmosphere. The reaction mixture was stirred at 100°C for 2 hours. The mixture was extracted (CH₂Cl₂/H₂O), dried, filtered and evaporated. The residue was used as intermediate (6).
b) Preparation of 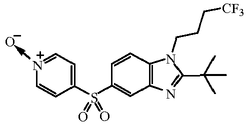 intermediate (7)
   Intermediate (6) (0.0023 mol) was dissolved in CHCl₃ (40 ml) at room temperature. 3-Chlorobenzenecarboperoxoic acid (0.0054 mol) was added slowly (exothermic reaction) and the mixture was stirred for 30 minutes. The reaction mixture was extracted twice with saturated NaHCO₃ and once with 1N NaOH. The organic layers were washed with water and dried (MgSO₄). The residue was purified by high-performance liquid chromatography using purification method B. The desired fractions were collected and the solvent was evaporated, yielding intermediate (7).
c) Preparation of 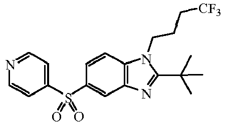 compound (4)
   Intermediate (7) (0.0006 mol) was dissolved in acetic acid (10 ml) and iron powder (0.0057 mol) was added. The reaction mixture was stirred at 60°C for 2 hours The reaction was cooled, evaporated and extracted (CH₂Cl₂/NaHCO₃). The organic layer was dried, filtered and evaporated, yielding compound (4).

### Example B.5

Preparation of 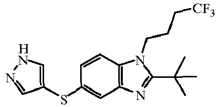 compound (8)
Dioxane (10ml) was added to a mixture of 1-benzoyl-4-iodo-1H-pyrazole (0.003 mol) and Cs₂CO₃ (0.00235 mol). The reaction mixture was degassed by applying alternating a nitrogen atmosphere and vacuum. A mixture of intermediate (5) (0.0023 mol) in dioxane (10 ml) was degassed by applying alternating a nitrogen atmosphere and vacuum. Pd₂(dba)₃ (0.1 g) and Xantphos (0.13 g) were added and the reaction mixture was degassed by applying alternating a nitrogen atmosphere and vacuum. The mixture was stirred at 70°C overnight. The mixture was cooled, water (150 ml) was added and the mixture was extracted 2 times with CH₂Cl₂ (150 ml). The combined organic layers were dried (MgSO₄), filtered and concentrated on the rotavap. The obtained residue was purified by high-performance liquid chroma-tography by purification method A. The desired product fraction was collected as compound (8).

### Example B.6

Preparation of 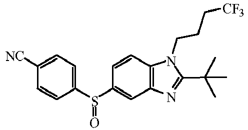 compound (7)
A mixture of compound (9) (0.0024 mol), 3-chlorobenzenecarboperoxoic acid (0.95 g; 70%) in CHCl₃ (20 ml) was shaken for 30 minutes at room temperature. The reaction mixture was washed with 1N NaOH (2 x 15 ml), with water (15 ml) and was then filtered over an Isolute™ filter and concentrated under a stream of nitrogen. The obtained residue was purified via reversed phase high-performance liquid chromatography. The desired product fraction was collected as compound (7).

### Example B.7

a) Preparation of 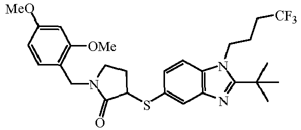 intermediate (12)
   To a mixture of intermediate (11) (0.009 mol) and intermediate (5) (0.003 mol) in DMF (15 mL) was added K₂CO₃ (0.0045 mol). The reaction mixture was stirred at 60°C for 1 hour. Then NaBH4 (0.0009 mol) was added and stirring was continued at 60°C for 1 hour. The mixture was concentrated and the residue was partitioned between ethylacetate and water. The organic layer was dried (MgSO4), filtered and evaporated. The residue was purified by column chromatography using on silica using a mixture of CH₂Cl₂ / methanol(7N NH₃) from 100:0 to 98:2 as eluent. The product fractions were collected and evaporated, yielding intermediate (12).
b) Preparation of 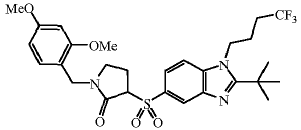 intermediate (13)
   Intermediate (12) (0.0011 mol) was dissolved in CH₂Cl₂ (30 ml) at room temperature. 3-Chlorobenzenecarboperoxoic acid (0.0033 mol) was added portionwise and the mixture was stirred for 30 min. The reaction mixture was washed with water and with 1N NaOH. The organic layer was dried (MgSO₄), filtered, and the solvent was evaporated, yielding intermediate (13).
c) Preparation of 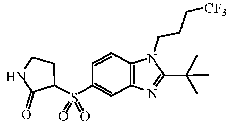 compound (16)
   A mixture of intermediate (13) (0.007 mol) in CF₃COOH (5 mL) was heated in a microwave for 20 min. at 100°C. The solvent was evaporated and the residue was partitioned between ethylacetate and aqeuous NaHCO3 solution. Then the organic layer was dried (MgSO4) and the solvent was evaporated. The residue was purified by column chromatography using CH₂Cl₂ / Methanol(7N NH3) from 100:0 to 98:2 as eluent. The product fractions were collected and evaporated. The residue was crystallized from methanol and diisopropylether. The solid was filtered off, washed and dried, yielding compound (16).

### Example B.8

a) Preparation of 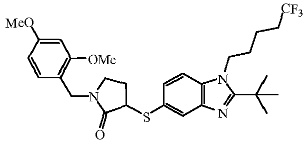 intermediate (14)
   To a mixture of intermediate (11) (0.002 mol) and intermediate (9) (0.001 mol) in DMF (15 mL) was added K₂CO₃ (0.0015 mol). The reaction mixture was stirred at 60°C for 1 hour. Then NaBH₄ (0.0003 mol) was added and stirring was continued at 60°C for 1 hour. The mixture was concentrated and the residue was partitioned between ethylacetate and water. The organic layer was dried (MgSO4), filtered and evaporated, yielding intermediate (14).
c) Preparation of 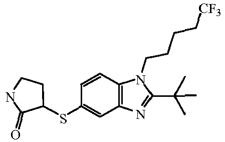 compound (17)
   A mixture of intermediate (14) (0.004 mol) in CF₃COOH (5 mL) was heated in a microwave for 5 min. at 100°C. The solvent was evaporated and the residue was partitioned between ethylacetate and aqeuous NaHCO3 solution. Then the organic layer was dried (MgSO4) and the solvent was evaporated. The obtained residue was purified by high-performance liquid chromatography by purification method A. The desired product fraction was collected as compound (17).

### Example B.9

Preparation of 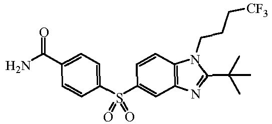 compound (18)
A mixture of compound (6) (0.0002 mol) in H₂SO₄ (3 mL) was stirred at room temperature for 20 hours. The mixture was poured on icewater (100 mL) and basified with aqueous NH₃ solution. The aqueous layer was extracted with CH₂Cl₂, the organic layer was dried (MgSO4) and the solvent was evaporated. The obtained residue was purified by high-performance liquid chromatography by purification method B. The desired product fraction was collected as compound (18).

### Example B.10

a) Preparation of 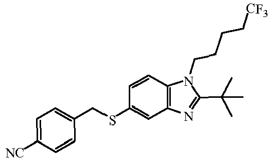 intermediate (15)
   A mixture of intermediate (9) (0.01 mol), 4-cyanobenzylbromide (0.015 mol) and K2CO3 (0.015 mol) in DMF (100 mL) was stirred at 60°C for 16 h. The reaction mixture was concentrated under reduced pressure, and the residue was partitioned between ethylacetate and water. The organic layer was dried (MgSO4), flitered and the solvent was evaporated. The residue was purified by column chromatography on silica gel using CH₂Cl₂ as eluent. The product fractions were collected and evaporated, yielding intermediate (15).
b) Preparation of 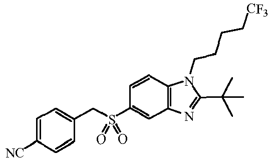 compound (19)
   Intermediate (12) (0.0069 mol) was dissolved in CH₂Cl₂ (100 ml) at room temperature. 3-Chlorobenzenecarboperoxoic acid (0.0172 mol) was added portionwise (exothermic reaction) and the mixture was stirred for 1 hour. The reaction mixture was washed with water and with 1N NaOH. The organic layer was dried (MgSO₄), filtered, and the solvent was evaporated. The residue was triturated with DIPE and 2-propanol, yielding compound (19).

### Example B.11

Preparation of 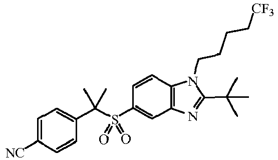 compound (20)
A mixture of compound (19) (0.0021 mol) in THF (50 mL) was stirred at 0°C under a nitrogen atmosphere. A 1M solution of sodium bis(trimethylsilyl)amide in THF (7.3 mL) was added dropwise and the mixture was stirred at 0°C for 1 hour. Methyliodide (0.0314 mol) was added and the reaction mixture was stirred for 1 hour at room temperature. The solvent was removed under reduced pressure and the residue was taken up in CH₂Cl₂ and washed with water. The organic layer was dried (MgSO4), filtered, and the solvent was evaporated. The obtained residue was solidified in DIPE and 2-propanol, yielding compound (20).

### Example B.12

Preparation of 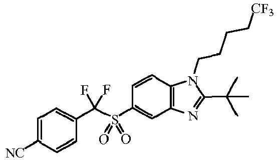 compound (21)
A mixture of compound (19) (0.0003 mol) in THF (50 mL) was stirred at 0°C under a nitrogen atmosphere. A 1M solution of sodium bis(trimethylsilyl)amide in THF (1.3 mL) was added dropwise and the mixture was stirred at 0°C for 1 hour. N-fluoro-dibenzenesulfonimide (0.0013 mol) was added and the reaction mixture was stirred at 0°C for 1 hour.The solvent was removed under reduced pressure and the residue was taken up in CH₂Cl₂ and washed with water. The organic layer was dried (MgSO4), filtered, and the solvent was evaporated. The obtained residue was purified by high-performance liquid chromatography by purification method B. The desired product fraction was collected as compound (21).

Table F-1 lists the compounds that were prepared according to one of the above Examples.

**Table F-1 (*) : compound (1) is provided only for the purpose of illustration**

| | |
|---|---|
| 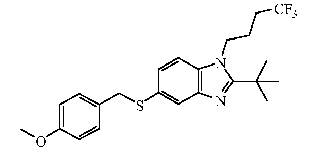 | 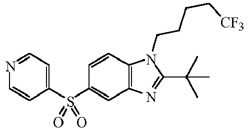 |
| Co. No.1 (*); Ex. B.1 | Co. No.13; Ex. B.3 |
| 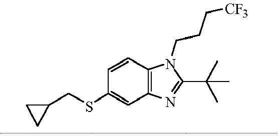 | 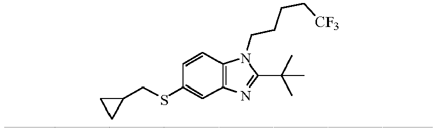 |
| Co. No.2; Ex. B.2 | Co. No.14; Ex. B.2 |
| 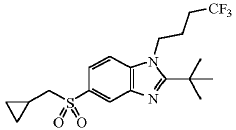 | 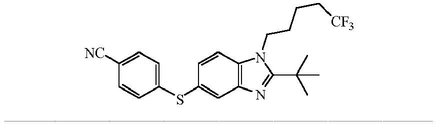 |
| Co. No.3; Ex. B.3 | Co. No.15; Ex. B.5 |
| 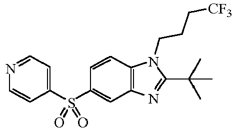 | 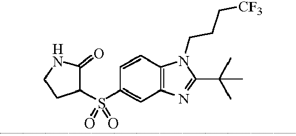 |
| Co. No.4; Ex. B.4 | Co. No.16; Ex. B.7 |
| 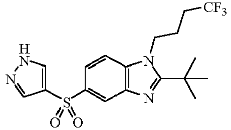 | 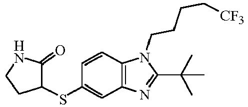 |
| Co. No.5; Ex. B.3 | Co. No.17; Ex. B.8 |
| 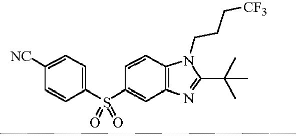 | 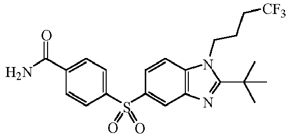 |
| Co. No.6; Ex. B.3 | Co. No. 18; Ex. B.9 |
| 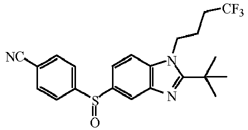 | 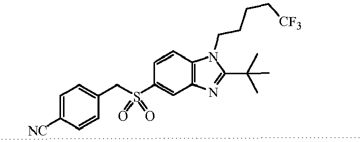 |
| Co. No.7; Ex. B.6 | Co. No. 19; Ex. B.10 |
| 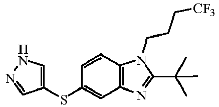 | 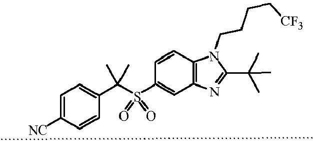 |
| Co. No.8; Ex. B.5 | Co. No.20; Ex. B.11 |
| 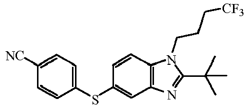 | 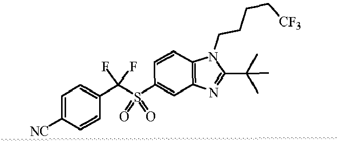 |
| Co. No.9; Ex. B.5 | Co. No.21; Ex. B.12 |
| 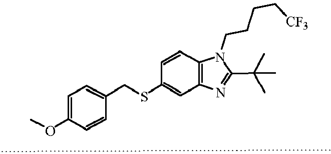 | 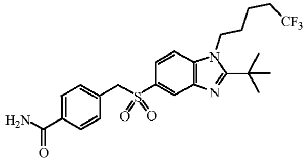 |
| Co. No. 10; Ex. B.1 | Co. No.22; Ex. B.9 |
| 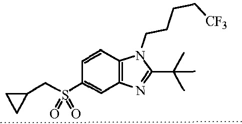 | 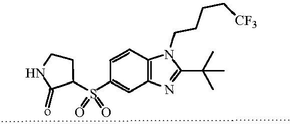 |
| Co. No.11; Ex. B.3 | Co. No.23; Ex. B.3 |
| 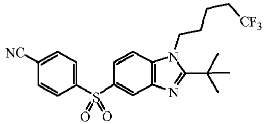 | |
| Co. No.12; Ex. B.4 | |

### C. Compound identification

### C. Analytical Part

### C.1 Melting Points

For a number of compounds, melting points (m.p.) were determined with a DSC823e (Mettler-Toledo). Melting points were measured with a temperature gradient of 30 °C/minute. The reported values are peak values. Maximum temperature was 400°C. Values are obtained with experimental uncertainties that are commonly associated with this analytical method.

| **Co. Nr.** | **m.p. (°C)** | **Co. Nr.** | **m.p. (°C)** |
|---|---|---|---|
| 1 | 143.2 | 16 | 208.7 |
| 4 | 155.4 | 18 | 207.8 |
| 5 | 220.8 | 19 | 177.4 |
| 6 | 165.1 | 20 | 187.6 |
| 12 | 180.1 | 22 | 222.8 |
| 13 | 147.6 | | |

### C.2 LCMS

### LCMS General procedure A

The HPLC measurement was performed using an Alliance HT 2790 (Waters) system comprising a quaternary pump with degasser, an autosampler, a column oven (set at 40 °C, unless otherwise indicated), a diode-array detector (DAD) and a column as specified in the respective methods below. Flow from the column was split to a MS spectrometer. The MS detector was configured with an electrospray ionization source. Mass spectra were acquired by scanning from 100 to 1000 in 1 second using a dwell time of 0.1 second. The capillary needle voltage was 3 kV and the source temperature was maintained at 140 °C. Nitrogen was used as the nebulizer gas. Data acquisition was performed with a Waters-Micromass MassLynx-Openlynx data system.

### LCMS General procedure B

The LC measurement was performed using an Acquity UPLC (Waters) system comprising a binary pump, a sample organizer, a column heater (set at 55 °C), a diode-array detector (DAD) and a column as specified in the respective methods below. Flow from the column was split to a MS spectrometer. The MS detector was configured with an electrospray ionization source. Mass spectra were acquired by scanning from 100 to 1000 in 0.18 seconds using a dwell time of 0.02 seconds. The capillary needle voltage was 3.5 kV and the source temperature was maintained at 140 °C. Nitrogen was used as the nebulizer gas. Data acquisition was performed with a Waters-Micromass MassLynx-Openlynx data system.

### LCMS - Procedure 1

In addition to general procedure A: Reversed phase HPLC was carried out on an Xterra MS C18 column (3.5 µm, 4.6 x 100 mm) with a flow rate of 1.6 ml/min. Three mobile phases (mobile phase A: 95% 25 mM ammoniumacetate + 5 % acetonitrile; mobile phase B: acetonitrile; mobile phase C: methanol) were employed to run a gradient condition from 100 % A to 1 % A, 49 % B and 50 % C in 6.5 minutes, to 1 % A and 99 % B in 1 minute and hold these conditions for 1 minute and reequilibrate with 100 % A for 1.5 minutes. An injection volume of 10 µl was used. Cone voltage was 10 V for positive ionization mode and 20 V for negative ionization mode.

### LCMS - Procedure 2

In addition to general procedure B: Reversed phase UPLC (Ultra Performance Liquid Chromatography) was carried out on a bridged ethylsiloxane/silica hybrid (BEH) C18 column (1.7 µm, 2.1 x 50 mm; Waters Acquity) with a flow rate of 0.8 ml/min. Two mobile phases (mobile phase A: 0.1 % formic acid in H₂O/methanol 95/5; mobile phase B: methanol) were used to run a gradient condition from 95 % A and 5 % B to 5 % A and 95 % B in 1.3 minutes and hold for 0.2 minutes. An injection volume of 0.5 µl was used. Cone voltage was 10 V for positive ionization mode and 20 V for negative ionization mode.

**Table : Analytical data-Retention time (Rₜ in minutes), (MH)⁺ peak and LCMS procedure.**

| **Co. Nr.** | **Rₜ** | **(MH)⁺** | **LCMS Procedure** |
|---|---|---|---|
| **3** | 5.71 | 403 | 1 |
| **9** | 1.35 | 418 | 2 |
| **10** | 1.28 | 451 | 2 |
| **11** | 1.24 | 417 | 2 |
| **15** | 1.14 | 432 | 2 |
| **17** | 0.99 | 414 | 2 |
| **21** | 1.36 | 514 | 2 |
| **23** | 1.12 | 446 | 2 |

### D. Pharmacological examples

### D.1 Inhibition of cAMP in response to activation of the human CB1 and CB2 receptors

Functional activity of the test compounds was assessed by measuring their potency to inhibit forskolin-activated cAMP production upon activation of the human CB1 (hCB1) or human CB2 (hCB2) receptor through homogenous time resolved fluorescence (HTRF) assays.

CHO-K1 cells stably transfected with either hCB1 or hCB2 were grown up to 80-90% confluence in T175 Falcon flasks in DMEM/NUT MIX F-12 culture medium complemented with 2% Solution A (5.10⁶ IU/1 penicillin G, 5 g/l streptomycin sulphate, 5.5 g/l pyruvate, 14.6 g/l L-glutamine, 1M NaOH) and 10% foetal calf serum. Before the experiments, medium was removed, cells were washed with PBS / EDTA (140 mM NaCl, 1 mM Na₂-EDTA, 8 mM Na₂HPO₄.2H₂O, 8.5 mM KH₂PO₄, 2.7 mM KCl, 21 mM glucose), resuspended in stimulation buffer (HBSS 1x, IBMX 1mM, Hepes 5mM, MgCl2 10mM, BSA 0.1%, pH 7.4). Cells were diluted to a concentration of 8.10⁵ cell/ml for hCB1 experiments and 10⁶ cells/ml for hCB2 experiments. Assays were performed using the cAMP Dynamic HTRF kit (CIS bio international, France) according to the recommendations of the manufacturer.

For CB1, each well of a 96 flat bottom black polystyrene assay plate (Costar) was filled with 25 µl stimulation buffer containing 6 µM forskolin and either test compound (in 2% DMSO), 2% DMSO or 2 µM CP55490 (in 2% DMSO). Then, 25 µl of the diluted cells was added (20,000 cells/well). After 30 minutes incubation in dark at room temperature, 25 µl cAMP-XL665 and 25 µl anti-cAMP cryptate (both at a final dilution of 1/80) was added to the cells.

For CB2, each well of a 384 flat bottom black polystyrene assay plate (Costar) was filled with 10 µl stimulation buffer containing 15 µM forskolin and either test compound (in 3% DMSO), 3% DMSO or 10 µM Win55212-2 (in 3% DMSO). Then, 20 µl of the diluted hCB2-CHO-K1 cells was added (20,000 cells/well). After 30 minutes incubation in dark at room temperature, 10 µl cAMP-XL665 and 10 µl anti-cAMP cryptate (both at a final dilution of 1/100) was added to the cells.

After equilibration of the reaction mixtures for 1 to 24 hours in dark at room temperature, fluorescence was measured at 665 nm and 620 nm using a Discovery microplate fluorescence counter (Perkin Elmer), and the signal ratio of 665 nm / 620 nm was calculated. The signal ratios of the test compounds were expressed relative to the signal ratios of the DMSO control (maximal signal ratio, no inhibition of cAMP) and CP55490 or WIN55212-2 for hCB1 and hCB2, respectively (minimal signal ratio, maximal inhibition of cAMP). From the dose response curves generated for each test compound, the dose at which 50% of the maximal inhibition of cAMP level is observed (EC₅₀, expressed in the Tables as pEC₅₀ = -log(EC₅₀) values) and the level of inhibition reached with 10 µM of the test compound compared to CP55490 (for hCB1) or WIN55212-2 (for hCB2) was calculated.

**Table D.1 : pEC50 values for CB-1 and CB-2 agonism**

| | CB-1 | CB-2 | | CB-1 | CB-2 |
|---|---|---|---|---|---|
| Co. No. | pEC50 | pEC50 | Co. No. | pEC50 | pEC50 |
| 1 | < 5.0 | 8.3 | 13 | 6.7 | 8.8 |
| 3 | 6.5 | 8.9 | 14 | 8.0 | 8.7 |
| 4 | 5.6 | 9.4 | 15 | 8.3 | 8.8 |
| 5 | 5.9 | 9.1 | 16 | 5.4 | 8.6 |
| 6 | 7.1 | 9.4 | 18 | 5.7 | 8.5 |
| 7 | <5.0 | 8.6 | 19 | < 5.0 | 8.7 |
| 8 | < 5.0 | 9.2 | 20 | 5.7 | 8.8 |
| 9 | 7.6 | 9.0 | 21 | 7.1 | 8.5 |
| 10 | < 5.0 | 8.2 | 22 | 7.1 | 8.7 |
| 11 | 7.5 | 8.8 | 23 | 5.8 | 8.3 |
| 12 | 7.6 | 9.0 | | | |

## Claims

1. Compound of formula (I) 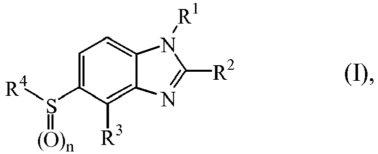 including any stereochemically isomeric form thereof, wherein
n is an integer 0, 1 or 2;
R¹ is n-trifluorobutyl or n-trifluoropentyl;
R² is C₁₋₆alkyl;
R³ is hydrogen;
R⁴ is C₁₋₈alkyl substituted with C₃₋₈cycloalkyl;
C₁₋₈alkyl substituted with 1, 2 or 3 substituents each independently selected from halo, C₁₋₈alkyl, or aryl;
heterocyclyl;
aryl; or
heteroaryl;
wherein
heterocyclyl is selected from pyrrolidinonyl;
aryl is phenyl substituted with 1 substituent selected from C₁₋₄alkyloxy, cyano or R⁷-carbonyl; wherein R⁷ is amino;
heteroaryl is selected from unsubsituted pyrazolyl or unsubstituted pyridinyl;
or a pharmaceutically acceptable acid addition salt thereof;
provided that 2-tert-butyl-5-[(4-methoxybenzyl)sulfanyl]-1-(4,4,4-trifluorobutyl)-1H-benzimidazole is not included.

2. The compound as claimed in claim 1 wherein R² is *tert*-butyl.

3. The pharmaceutical composition comprising a pharmaceutically acceptable carrier and a therapeutically active amount of a compound as claimed in any of claims 1 to 2.

4. A process for preparing a pharmaceutical composition as claimed in claim 3 wherein a therapeutically active amount of a compound as claimed in any of claims 1 to 2 is intimately mixed with a pharmaceutically acceptable carrier.

5. The compound as claimed in any of claims 1 to 2 for use as a medicine.

6. The compound as claimed in any of claims 1 to 2 for use in the treatment of atherosclerosis, hypertension, myocardial ischemia, hyperalgesia, neuropathic pain, peripheral pain, visceral pain, inflammatory pain, thermal hyperalgesia, nociceptive pain, fibromyalgia, chronic low back pain, dental pain, inflammation, oedema, bladder inflammation, neuroinflammatory diseases, immune system disorders, autoimmune diseases, multiple sclerosis, rheumatoid arthritis, gastrointestinal disorders, intestinal motility disorders, irritable bowel syndrome (IBS), inflammatory bowel disease (IBD), Crohn's disease, chronic liver injury (cirrhosis), cancer, prostate cancer, cancer pain, glioma, allergy, nausea and vomiting, asthma, chronic obstructive pulmonary diseases, psoriasis, epilepsy, and osteoporosis.

7. A process for preparing a compound of formula (I-a), defined as a compound of formula (I) as claimed in claim 1 wherein n is 0, by reacting an intermediate (II) with an intermediate (III), wherein L is a leaving group in the presence of a suitable base in a reaction-inert solvent; 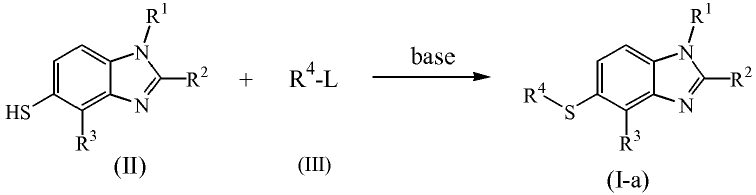 wherein R¹, R², R³ and R⁴ are defined as in claim 1;
or; if desired; a compound of formula (I-a) is converted into a pharmaceutically acceptable acid addition salt, or conversely, an acid addition salt of a compound of formula (I-a) is converted into a free base form with alkali; and, if desired, preparing stereochemically isomeric forms thereof

8. A process for preparing a compound of formula (I-b), defined as a compound of formula (I) as claimed in claim 1 wherein n is 1, by S-oxidizing a compound of formula (I-a), wherein R¹, R², R³ and R⁴ are as defined in claim 1, with an oxidizing agent; 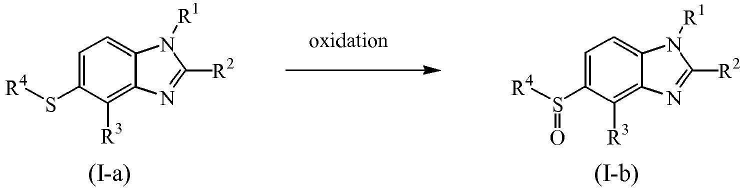 or; if desired; a compound of formula (I-b) is converted into a pharmaceutically acceptable acid addition salt, or conversely, an acid addition salt of a compound of formula (I-b) is converted into a free base form with alkali; and, if desired, preparing stereochemically isomeric forms thereof

9. A process for preparing a compound of formula (I-c), defined as a compound of formula (I) as claimed in claim 1 wherein n is 1, by S-oxidizing a compound of formula (I-a), wherein R¹, R², R³ and R⁴ are as defined in claim 1, with an oxidizing agent; 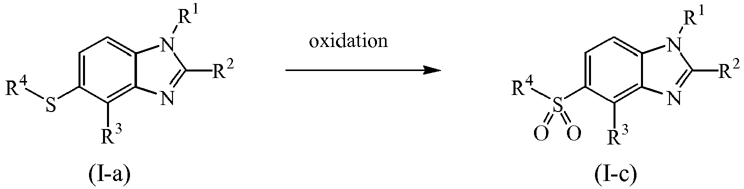 or; if desired; a compound of formula (I-c) is converted into a pharmaceutically acceptable acid addition salt, or conversely, an acid addition salt of a compound of formula (I-c) is converted into a free base form with alkali; and, if desired, preparing stereochemically isomeric forms thereof

## Patentansprüche

1. Verbindung der Formel (I) 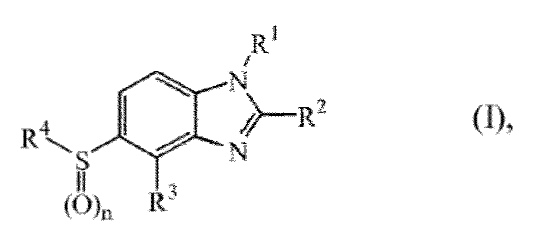 einschließlich aller stereochemisch isomeren Formen davon, wobei
n für eine ganze Zahl 0, 1 oder 2 steht,
R¹ für n-Trifluorbutyl oder n-Trifluorpentyl steht,
R² für C₁₋₆-Alkyl steht,
R³ für Wasserstoff steht,
R⁴ für durch C₃₋₈-Cycloalkyl substituiertes C₁₋₈-Alkyl,
durch 1, 2 oder 3 jeweils unabhängig voneinander aus Halogen, C₁₋₈-Alkyl und Aryl ausgewählte Substituenten substituiertes C₁₋₈-Alkyl,
Heterocyclyl
Aryl oder
Heteroaryl steht,
wobei
Heterocyclyl aus Pyrrolidinonyl ausgewählt ist,
Aryl für durch einen aus C₁₋₄-Alkyloxy, Cyano und R⁷-Carbonyl ausgewählten Substituenten substituiertes Phenyl steht, wobei R⁷ für Amino steht,
Heteroaryl aus unsubstituiertem Pyrazolyl und unsubstituiertem Pyridinyl ausgewählt ist,
oder ein pharmazeutisch unbedenkliches Säureadditionssalz davon,
mit der Maßgabe, dass 2-tert.-Butyl-5-[(4-methoxybenzyl)sulfanyl]-1-(4,4,4-trifluorbutyl)-1H-benzimidazol ausgeschlossen ist.

2. Verbindung nach Anspruch 1, wobei R² für *tert.-*Butyl steht.

3. Pharmazeutische Zusammensetzung, umfassend einen pharmazeutisch unbedenklichen Träger und eine therapeutisch wirksame Menge einer Verbindung nach Anspruch 1 oder 2.

4. Verfahren zur Herstellung einer pharmazeutischen Zusammensetzung nach Anspruch 3, bei dem man eine therapeutisch wirksame Menge einer Verbindung nach Anspruch 1 oder 2 innig mit einem pharmazeutisch unbedenklichen Träger mischt.

5. Verbindung nach Anspruch 1 oder 2 zur Verwendung in der Medizin.

6. Verbindung nach Anspruch 1 oder 2 zur Verwendung bei der Behandlung von Atherosklerose, Hypertonie, myokardialer Ischämie, Hyperalgesie, neuropathischen Schmerzen, peripheren Schmerzen, viszeralen Schmerzen, entzündlichen Schmerzen, thermaler Hyperalgesie, nozizeptiven Schmerzen, Fibromyalgie, chronischen Schmerzen im unteren Rücken, Zahnschmerzen, Entzündung, Ödem, Blasenentzündung, neuroinflammatorischen Krankheiten, Erkrankungen des Immunsystems, Autoimmunkrankheiten, multipler Sklerose, rheumatoider Arthritis, gastrointestinalen Erkrankungen, Störungen der intestinalen Motilität, Reizkolon (Irritable Bowel Syndrome, IBS), entzündlicher Darmerkrankung (Inflammatory Bowel Disease, IBD), Morbus Crohn, chronischer Leberschädigung (Zirrhose), Krebs, Prostatakrebs, Krebsschmerzen, Gliom, Allergie, Übelkeit und Erbrechen, Asthma, chronischen obstruktiven Atemwegserkrankungen, Psoriasis, Epilepsie und Osteoporose.

7. Verfahren zur Herstellung einer Verbindung der Formel (I-a), die als eine Verbindung der Formel
(I) nach Anspruch 1 definiert ist, in welcher n für 0 steht, durch Umsetzen eines Zwischenprodukts
(II) mit einem Zwischenprodukt (III), wobei L für eine Abgangsgruppe steht, in Gegenwart einer geeigneten Base in einem reaktionsinertem Lösungsmittel 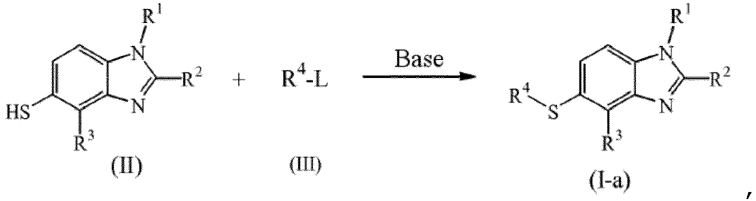 wobei R¹, R², R³ und R⁴ wie in Anspruch 1 definiert sind,
oder, falls gewünscht, eine Verbindung der Formel (I-a) in ein pharmazeutisch unbedenkliches Säureadditionssalz umgewandelt wird oder umgekehrt ein Säureadditionssalz einer Verbindung der Formel (I-a) mit Alkali in eine freie Basenform umgewandelt wird und, falls gewünscht, Herstellung stereochemisch isomerer Formen davon.

8. Verfahren zur Herstellung einer Verbindung der Formel (I-b), die als eine Verbindung der Formel (I) nach Anspruch 1 definiert ist, in welcher n für 1 steht, durch S-Oxidieren einer Verbindung der Formel (I-a), wobei R¹, R², R³ und R⁴ wie in Anspruch 1 definiert sind, mit einem Oxidationsmittel 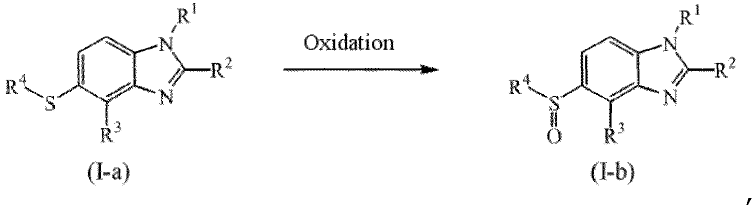 oder, falls gewünscht, eine Verbindung der Formel (I-b) in ein pharmazeutisch unbedenkliches Säureadditionssalz umgewandelt wird oder umgekehrt ein Säureadditionssalz einer Verbindung der Formel (I-b) mit Alkali in eine freie Basenform umgewandelt wird und, falls gewünscht, Herstellung stereochemisch isomerer Formen davon.

9. Verfahren zur Herstellung einer Verbindung der Formel (I-c), die als eine Verbindung der Formel (I) nach Anspruch 1 definiert ist, in welcher n für 1 steht, durch S-Oxidieren einer Verbindung der Formel (I-a), wobei R¹, R², R³ und R⁴ wie in Anspruch 1 definiert sind, mit einem Oxidationsmittel 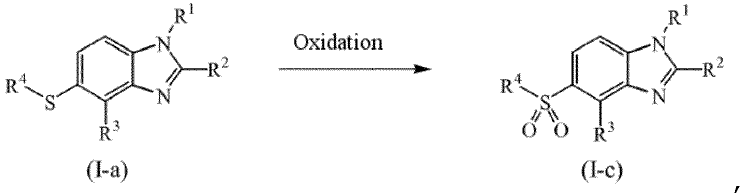 oder, falls gewünscht, eine Verbindung der Formel (I-c) in ein pharmazeutisch unbedenkliches Säureadditionssalz umgewandelt wird oder umgekehrt ein Säureadditionssalz einer Verbindung der Formel (I-c) mit Alkali in eine freie Basenform umgewandelt wird und, falls gewünscht, Herstellung stereochemisch isomerer Formen davon.

## Revendications

1. Composé de formule (I) 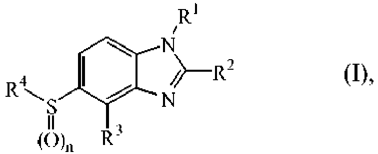
Comprenant une forme d'isomère stéréochimique de celui-ci, où n est un entier égal à 0, 1 ou 2 ;
R¹ est n-trifluorobutyle ou n-trifluoropentyle ;
R² est alkyle en C₁₋₆ ;
R³ est hydrogène ;
R⁴ est alkyle en C₁₋₈ substitué par cycloalkyle en C₁₋₈ ;
alkyle en C₁₋₈ substitué par 1, 2 ou 3 substituants chacun indépendamment choisi parmi halogéno, alkyle en C₁₋₈, ou aryle ;
hétérocyclyle ;
aryle ; ou
hétéroaryle ; où
hétérocyclyle est choisi parmi pyrrolidinonyle ;
aryle est phényle substitué par 1 substituant choisi parmi alkyloxy en C₁₋₄, cyano ou R⁷-carbonyle ; où R⁷ est amino ;
hétéroaryle est choisi parmi pyrazolyl non substitué ou pyridinyle non substitué ; ou un sel d'addition d'acide pharmaceutiquement acceptable de celui-ci ; à condition que le 2-tert-butyl-5-[(4-methoxybenzyl)sulfanyl]-1-(4,4,4-trifluorobutyl)-1H-benzimidazole ne soit pas inclus.

2. Composé selon la revendication 1 dans lequel R² est *tert*-butyle.

3. Composition pharmaceutique comprenant un véhicule pharmaceutiquement acceptable et une quantité thérapeutiquement active d'un composé selon l'une quelconque des revendications 1 à 2.

4. Procédé de préparation d'une composition pharmaceutique selon la revendication 3 dans lequel une quantité thérapeutiquement active d'un composé selon l'une quelconque des revendications 1 à 2 est intimement mélangé avec un véhicule pharmaceutiquement acceptable.

5. Composé selon l'une quelconque des revendications 1 à 2 pour utilisation en tant que médicament.

6. Composé selon l'une quelconque des revendications 1 à 2 pour utilisation dans le traitement de l'athérosclérose, l'hypertension, l'ischémie cardiaque, l'hyperalgésie, la douleur neuropathique, la douleur périphérique, la douleur viscérale, la douleur inflammatoire, l'hyperalgésie thermique, la douleur nociceptive, la fibromyalgie, la douleur lombaire chronique, la douleur dentaire, l'inflammation, l'oedème, une inflammation de la vessie, des maladies neuro-inflammatoires, des troubles du système immunitaire, des maladies auto-immunes, la sclérose en plaques, la polyarthrite rhumatoïde, des troubles gastro-intestinaux, des troubles de motilité intestinale, un syndrome abdominal inflammatoire, une maladie abdominale inflammatoire, la maladie de Crohn, une lésion hépatique chronique (cirrhose), un cancer, un cancer de la prostate, une douleur cancéreuse, un gliome, une allergie, des nausées et des vomissements, l'asthme, des bronchopneumopathies chroniques obstructives, le psoriasis, l'épilepsie et l'ostéoporose.

7. Procédé de préparation d'un composé de formule (I-a), défini comme étant un composé de formule (I) selon la revendication 1 dans lequel n est 0, par réaction d'un intermédiaire (II) avec un intermédiaire (III), où L est un groupe partant en présence d'une base adaptée dans un solvant inerte à la réaction ; 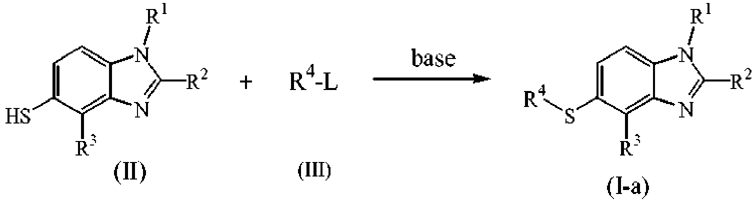
où R¹, R², R³ et R⁴ sont tels que définis dans la revendication 1 ;
ou, le cas échéant ; un composé de formule (I-a) est converti en sel d'addition d'acide pharmaceutiquement acceptable, ou inversement, un sel d'addition d'acide d'un composé de formule (I-a) est converti sous forme de base libre avec un alcali ; et, le cas échéant, préparation de de formes isomères stéréochimiques de celui-ci.

8. Procédé de préparation d'un composé de formule (I-b), défini comme étant un composé de formule (I) selon la revendication 1 où n est 1, par S-oxydation d'un composé de formule (I-a), où R¹, R², R³ et R⁴ sont tels que définis dans la revendication 1, avec un agent oxydant ; 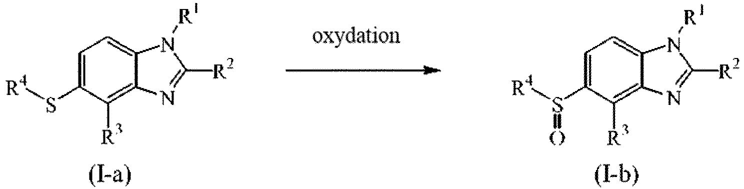 ou ; le cas échéant ; un composé de formule (I-b) est converti en sel d'addition d'acide pharmaceutiquement acceptable, ou inversement, un sel d'addition d'acide d'un composé de formule (I-b) est converti sous forme de base avec un alcali ; et, le cas échéant, préparation de formes isomères stéréochimiques de celui-ci.

9. Procédé de préparation d'un composé de formule (I-c), défini comme étant un composé de formule (I) selon la revendication 1 dans lequel n est 1, par S-oxydation d'un composé de formule (I-a), où R¹, R², R³ et R⁴ sont tels que définis dans la revendication 1, avec un agent oxydant ; 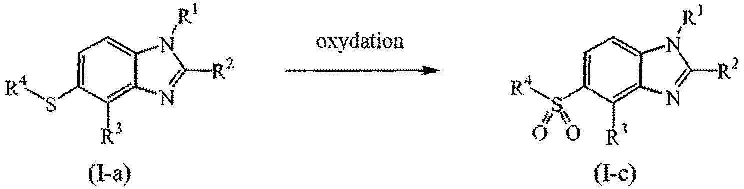 ou ; le cas échéant ; un composé de formule (I-c) est converti en sel d'addition d'acide pharmaceutiquement acceptable, ou inversement, un sel d'addition d'acide d'un composé de formule (I-c) est converti sous forme de base libre avec un alcali ; et, le cas échéant, préparation de formes isomères stéréochimiques de celui-ci.
